# EUROPEAN PATENT SPECIFICATION

(11) **EP 2 522 996 B1**
(45) Date of publication and mention of the grant of the patent: **06.04.2016**
(21) Application number: 12167359.4
(22) Date of filing: 09.05.2012
(51) Int. Cl.: G01N 33/04, G01N 33/569, G01N 33/53, G01N 33/558

(54) **Method for analysing aflatoxins in milk and milk by-products**
Verfahren zur Analyse von Aflatoxinen im Milch und Nebenprodukts aus Milch
Procédé d'analyse des aflatoxines dans le lait et dans des produits dérivés du lait

(30) Priority: 11.05.2011 IT MO20110109
(43) Date of publication of application: 14.11.2012
(73) Proprietor: GENERON S.r.l., 41126 Località S. Maria di Mugnano (MO) (IT)
(72) Inventor: Gatti, Marcello, 41126 Località S. Maria di Mugnano (MO) (IT)
(74) Representative: Brunacci, Marco

(56) References cited:
- EP-A1- 2 090 590
- WO-A1-94/15970
- WO-A1-03/006994
- WO-A1-2009/086621
- WO-A2-2007/094945
- GB-A- 2 193 809
- US-A1- 2008 014 582
- US-A1- 2009 081 808
- NGUNDI MIRIAM M ET AL: "Array biosensor for detection of ochratoxin A in cereals and beverages", ANALYTICAL CHEMISTRY, AMERICAN CHEMICAL SOCIETY, US, vol. 77, no. 1, 1 January 2005 (2005-01-01), pages 148-154, XP002430515, ISSN: 0003-2700, DOI: 10.1021/AC048957Y
- LAURA ANFOSSI ET AL: "Development of a quantitative lateral flow immunoassay for the detection of aflatoxins in maize", FOOD ADDITIVES & CONTAMINANTS: PART A, vol. 28, no. 2, 1 February 2011 (2011-02-01), pages 226-234, XP055199307, ISSN: 1944-0049, DOI: 10.1080/19440049.2010.540763
- Robert Salter ET AL: "FOOD CHEMICAL CONTAMINANTS Interlaboratory Study of the Charm ROSA Safe Level Aflatoxin M1 Quantitative Lateral Flow Test for Raw Bovine Milk", FOOD CHEMICAL CONTAMINANTS, 1 January 2006 (2006-01-01), XP055199310, [retrieved on 2015-06-30]

## Description

The present invention relates to a method for analysing M1 aflatoxins in milk and milk by-products.

The use is known of different systems for analysing aflatoxins, in particular type M1 aflatoxins.

A widely-used known technique is high-performance (or high pressure) liquid chromatography, more simply known by the English acronym HPLC (High Performance Liquid Chromatography).

The HPLC system is an instrumental chromatography and detection system on fluorimeter, wherein an extracted sample to be analyzed is introduced inside a high-pressure chromatographic column, in such a way as to separate any interferences from the M1 aflatoxin.

All the eluate exiting from the chromatographic column is analyzed in line and continuously by means of a fluorimetry detection system suitable for detecting the fluorescence of the toxin and therefore determining its presence or absence. The toxin is furthermore quantified by means of a comparison between the chromatogram area of the sample and the chromatogram area of a reference material undergoing the same treatment.

Generally speaking, to upgrade the quality of the extract and reduce the interfering co-extractives, the milk or milk by-products analyzed are purified with a further column of immunoaffinity before the injection into the HPLC system.

Such known system, however, has a number of drawbacks.

In particular, the duration of the analysis is considerable (generally speaking about 40 minutes), the cost of the system is high, inasmuch as based on the use of sophisticated equipment and, furthermore, the entire procedure is considerably complex.

An alternative known technique, used to analyze the M1 aflatoxin in milk, is that known by the English acronym ELISA (Enzyme-Linked ImmunoSorbent Assay).

ELISA is an immunological system which exploits the antigen/antibody reaction, wherein the antibodies or the antibody toxin conjugates are fixed to a plate split into a plurality of reaction wells (generally 48 or 96).

This known technique also has a number of drawbacks however.

Such technique in fact requires considerable manual skill on the part of operators.

Furthermore, the duration of the ELISA analysis for the determination of the M1 aflatoxin is considerable (about two hours).

Finally, the ELISA type systems necessarily require cold storage, at around 4-8°C.

Systems are furthermore known which require a so-called "lateral flow" type device for analyzing the quantity of M1 aflatoxin in milk.

In particular, such systems envisage the introduction of a sample of the liquid to be analyzed on the lateral-flow device and, subsequently, the positioning of the device inside an incubator.

In particular, the incubator is required to keep the lateral-flow device at a constant controlled temperature, generally speaking around 45 °C, for a predetermined time.

Once the incubation has terminated, the lateral-flow device can be fitted in a special scanning detector to detect the presence and/or the quantity of M1 aflatoxin.

These known lateral-flow type systems for determining the M1 aflatoxin also have a number of drawbacks.

First of all, they only allow performing an analysis of quantitative type, while such lateral-flow type systems do not allow any qualitative type analysis of the M1 aflatoxin by means of a simple visual inspection conducted in the absence of reading instruments.

Furthermore, such systems have a low pressure and are therefore of little use for accurate quantitative analyses, i.e., aimed at the precise measurement of the quantity of M1 aflatoxin in the milk or in the milk by-products.

The error of such systems, in fact, is not below 25% with respect to the expected reading.

In such systems of known type, the lateral-flow device is of the three-colour band type and therefore such systems are considerably complex to create from a technical viewpoint.

With such systems of known type, furthermore, it is necessary to work at controlled temperature and, therefore, additional devices must necessarily be used such as incubators or the like.

Document WO 2009/086621 A1 discloses the selection of specific types of DNA with the ability to directly bind mycotoxins, which serve as substitutes of the traditional antibodies for the production of systems for the purification of mycotoxins. However, the solution described does not work on the M1 aflatoxin.

EP 2 090 590 discloses the production and the use of a particular polyclonal antibody with high organic solvents tolerance, which can be used both for the detection and for the purification of toxins by immuno-affinity columns. In contrary, the present application does not use an immuno-affinity purification but uses the antibody to detect by chromatography on cellulose (lateral flow) the presence of the toxin M1. This application also does not foresee the use of antibodies with high organic solvents tolerance, since organic solvents are not used.

WO 2007/094945 discloses a particular configuration of immunoaffinity columns in which are suspended orbs able to purify the sample with reduction of the background noise signal, with a consequent reduction of the sensitivity of the detections that, again and again, can be applied to liquids so purified. Therefore, document WO 2007/094945 describes an enhancement of immunoaffinity columns of known type which has nothing to do with the present invention that, in a different way, does not use any phase of immunoaffinity purification.

Document WO 03/006994 discloses the detection of toxins by means of a variation of the polarization of the fluorescence signal. The scope of document WO 03/006994 is limited to cereal and nuts or agricultural products in general and WO 03/006994 does not disclose the use for M1 toxin in milk. In particular, the invention of D4 was developed for the determination of aflatoxins B1, B2, G1, and G2.

The main aim of the present invention is to provide a method for the M1 aflatoxin analysis in milk and milk by-products which can be used for quality analyses with respect to different threshold levels selectable by the user according to specific requirements, i.e., aimed at detecting the presence or absence of variable quantities of M1 aflatoxin in accordance with the selected sensitivity level or, alternatively, which can be used for precise and accurate quantity analyses, i.e., aimed at precisely measuring the quantity of M1 aflatoxin present.

Another object of the present invention is to provide a method for the M1 aflatoxin analysis in milk and milk by-products which can be easy to use. Another object of the present invention is to provide a method for the M1 aflatoxin analysis in milk and milk by-products which allows to operate quickly. Another object of the present invention is to provide a method for the M1 aflatoxin analysis in milk and milk by-products which allows to overcome the mentioned drawbacks of the state of the art within the ambit of a simple, rational, easy and effective to use as well as low cost solution.

The above objects are achieved by the present method for analysing M1 aflatoxins in milk and milk by-products, characterised by the fact that it comprises:
- at least a homogenization phase of a milk or milk by-product sample to be analysed;
- at least an extraction phase from said homogenized sample of an extract containing M1 aflatoxin;
- at least a competition phase between said extract and an antibody-dye conjugate for obtaining a mix to be analysed wherein the reaction between said M1 aflatoxin and said antibody-dye conjugate is at least partially completed;
- at least a chromatography phase of said mix by means of at least a chromatographic device of the type of a lateral-flow device or the like;
- at least a qualitative and/or quantitative reading phase of the results found by said chromatographic device, wherein said qualitative reading comprises the detection of the presence or not in said sample of a quantity of M1 aflatoxin higher than at least a pre-determined minimum threshold level, and wherein said quantitative reading comprises the measurement of the quantity of M1 aflatoxin present in said sample.

Other characteristics and advantages of the present invention will become more evident from the description of a preferred, but not sole, embodiment of a method for analysing M1 aflatoxins in milk and milk by-products, illustrated purely as an example but not limited to the annexed drawings in which:
Figure 1 is a general block diagram of the method according to the invention;
Figure 2 is an axonometric schematic view of the chromatographic device used in the method according to the invention;
Figures 3 and 4 illustrate the chromatographic device of figure 2 housed inside a suitable protection container.

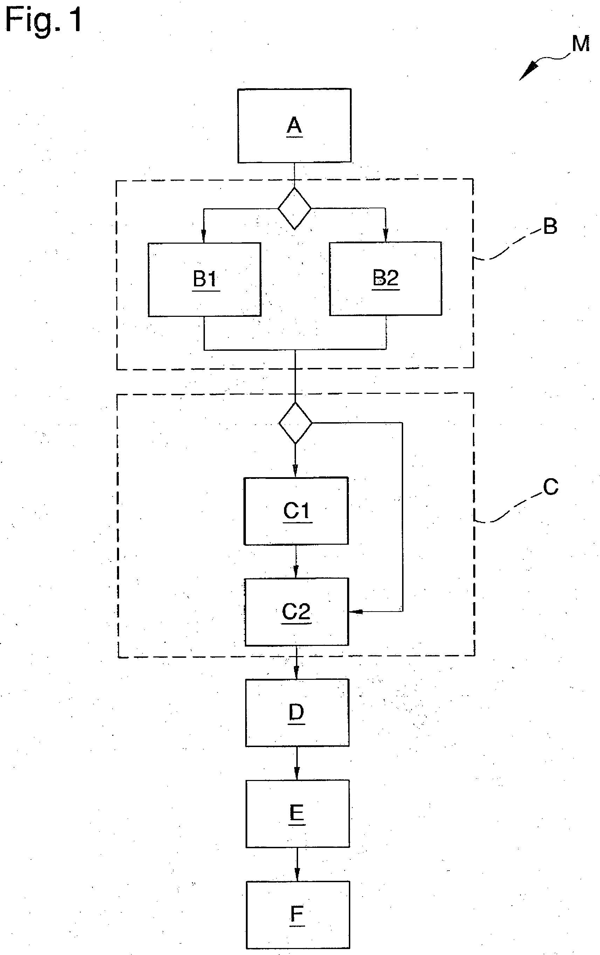

With particular reference to figure 1, globally indicated by M is a method for analysing M1 aflatoxins in milk and milk by-products.

In particular, the method according to the invention can be used to determine, with extremely high sensitivity, the quantity of M1 aflatoxin present in milk or in milk by-products.

Such level of sensitivity is fully compatible with legal requirements set at 50 ppb and here established by way of example at 10 ppb.

It is pointed out that by milk and milk by-products is typically meant: full-cream milk, high-quality milk, skimmed or partially-skimmed milk, UHT milk, buttermilk, dairy products and cheeses, milk-based mixes or which contain milk, powdered milk and concentrated milk.

The method M comprises the following descriptive phases.

First of all, the method M comprises a phase A of taking a sample of the milk or the milk by-product to be analyzed.

Such sample can be liquid or solid.

Subsequently, the method M comprises a homogenization phase B of the sample taken to be analyzed.

In particular, if the sample to be analyzed is a sample of a solid milk by-product, homogenization comprises a grinding phase B1 of the sample for a predetermined time, preferably 30 seconds.

Alternatively, if the sample to be analyzed is liquid, homogenization comprises a mixing phase B2 of the sample for a predetermined time, preferably 30 seconds.

Subsequently, the method M comprises an extraction phase C from the homogenized sample of an extract containing M1 aflatoxin.

In particular, if the sample to be analyzed is a solid milk by-product sample, the extraction comprises a dilution phase C1 of the homogenized sample by means of the addition of a predetermined quantity of water or aqueous solution. Preferably, the predetermined quantity of water or aqueous solution varies between 3 ml and 10 ml.

Subsequently, the extraction comprises a centrifugation phase C2 of the homogenized and diluted sample, preferably at 14,000 r.p.m. and for about 3 minutes.

Alternatively, if the sample to be analyzed is liquid, the extraction directly comprises the centrifugation phase C2 of the homogenized sample, preferably at 14,000 r.p.m. and for about 3 minutes.

The method M also comprises a competition phase D conducted separately between the obtained extract and an antibody-dye conjugate to obtain a mix to be analyzed.

Preferably, the dye used is colloidal gold.

The use of different dyes cannot however be ruled out such as, e.g., fluorescent dyes, Latex, etc.

Preferably, the antibodies which can be used are both polyclonal and monoclonal in rabbit, rat, mouse, sheep or other animal indistinctly. Advantageously, such antibodies are treated with a procedure suitable for overloading them with colloidal gold, so as to make them particularly distinguishable from the so-called background noise.

In particular, such treatment of the antibodies allows achieving greater sensitivity which permits detecting even the smallest traces of M1 aflatoxin, including in the case of very low concentration levels.

In practice, unlike common antibody treatments with colloidal gold which achieve a maximum level in the gold molecules per antibody molecule ratio, the M method according to the invention allows increasing the number of gold molecules per antibody molecule, conveying greater sensitivity for the detection of the M1 aflatoxin.

In particular, the system adopted for the production of the primary antibody conjugates with the colloidal gold allows mainly selecting molecular macro aggregates of colloidal gold of spherical or semi-spherical shape most suitable for being fixed to the primary antibody with minimum possible steric effects with respect to the other two known shapes of the colloidal gold, i.e., cylindrical or cubic.

This allows a more efficient insertion of the antibody colloidal gold particle on the antibodies, increasing the sensitivity of this normally obtainable using traditional methods.

It follows that such invention is able to double the sensitivity of the method M inasmuch as the detectable colour depends directly on the colour conveying molecules coupled with the antibody, i.e., on the colloidal gold molecules, and it further follows that the discrimination obtained, i.e., the capacity to distinguish even small signal variations, makes pointless the insertion of a third-colour band, as normally occurs in pre-existing systems.

In particular, the competition phase D comprises placing a predetermined quantity of the extract obtained inside an adequate plastic or glass container containing the antibody-gold conjugate and waiting for a predetermined waiting time at ambient temperature, so as to obtain a mix wherein the reaction between the M1 aflatoxin and the antibody-dye conjugate is at least partially completed. Preferably, the quantity of extract used varies between 60 µl and 280 µl.

The predetermined waiting time varies between 5 minutes and 25 minutes and preferably lasts 10 minutes.

In a preferred embodiment of the method M, the competition phase D comprises placing the pre-defined quantity of obtained extract inside a container containing the antibody-gold conjugate together with an organic or inorganic saline solution (polyethylene glycol and boron and/or citrate salts).

In particular, the antibody-gold conjugate and the organic and/or inorganic saline solution can be in liquid form or, alternatively, can both be preserved in lyophilized form inside a single container.

The competition phase D, and in particular the fact of having the reaction occur separately between the aflatoxin M1 and the antibody-colloidal gold conjugate in a separate container for a predetermined time, allows the anti aflatoxin M1 antibody fixed to the colloidal gold to bond with the aflatoxin M1 with the right reaction times, permitting a better reproducibility of the assay.

In a different way, in the known lateral-flow type systems, the competition reaction occurs directly during the chromatography phase on the lateral-flow device itself, and consequently the M1 aflatoxin and the conjugated antibody have much less time to react, thus providing less precise and therefore less reproducible data.

The use of an organic saline solution, in particular, allows reducing the effect of general abatement of the signal detectable on the chromatographic areas of the chromatographic device 1 caused by the milk and milk by-product matrices.

In fact, the use in particular of polyethylene glycols in a concentration variable between 1 and 10% with respect to the assay volume, together with salts such as boron and/or citrate permits almost completely restoring the signal lost due to the matrix, enabling the assay to recover the sensitivity lost due to the milk or milk by-product matrix.

Furthermore, the use of the antibody-gold conjugate and of the organic saline solution in lyophilized form makes it possible to stabilise the reactants and ensure their long life in full chemical activity.

It is known, in fact, that the removal of water by freeze drying and in vacuum (lyophilisation) reduces the chemical mobility of the substances and reduces water activity, preventing the growth of bacteria which would spoil the assay and consequently impede the common degradation reactions of the molecules themselves due to formation of intermediates with disadvantageous increase of the degree of entropy.

Subsequently, the method M comprises a chromatography phase E of the mix obtained from the competition phase D.

Advantageously, the chromatography is performed by means of a chromatography device of the type of a lateral-flow device or the like.

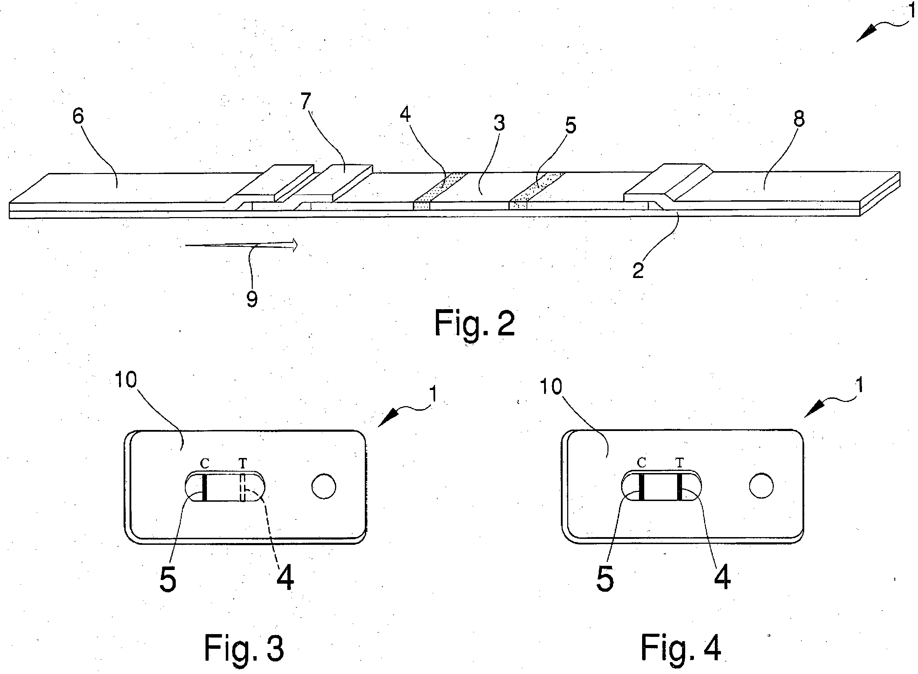

In particular, in a preferred embodiment, the chromatographic device used is the same as the lateral-flow type device shown in figure 2 and indicated altogether by the reference 1.

The chromatography phase E, in particular, comprises the introduction of the chromatography device 1 inside the container containing the mix for a predetermined waiting time which, preferably, varies between 5 minutes and 25 minutes.

Finally, the method M comprises a qualitative and/or quantitative reading phase F of the results detected by the chromatographic device 1.

In particular, by the term "qualitative reading" is meant the detection of the presence or not in the milk or milk by-product sample of a quantity of M1 aflatoxin above a predetermined threshold level.

The threshold level can be chosen at the time of realisation of the chromatographic device 1, thereby differing the type of measurement that can be made and, therefore, the type of product.

In particular, the threshold level can be set between 20 and 30 ppt, in compliance with current market requirements and standards applicable at European level.

By the term "quantitative reading", instead, is meant the measurement of the quantity of M1 aflatoxin in the milk or the milk by-product sample.

Preferably, the range of values that can be measured by means of quantitative reading is between 0 and 100 ppt.

Preferably, the reading is taken by introducing the chromatographic device 1 into a flat scanner of the conventional type.

Advantageously, on average the method M altogether lasts about 25-30 minutes. With reference to the preferred but not only embodiment shown in figure 2, the chromatographic device 1 comprises a support element 2 consisting of a strip of plastic material.

An analysis membrane 3 is fastened to the support element 2 by gluing or mechanical fastening and is suitable for performing the chromatographic analysis of the mix obtained from the competition phase D.

Preferably, the analysis membrane 3 consists of a strip with a substantially elongated shape made of cellulose, acetate or cellulose nitrate or other similar substances.

In particular, the analysis membrane 3 has a chromatographic test area made up of the line indicated in figure 2 with the reference 4, having reactants able, during their passage, to fix the antibody molecules which have not reacted with the extract or which have partially reacted with the extract during the competition phase D.

Preferably, the reactants present in the chromatographic test area 4 comprise a toxin protein conjugate able to fix primary antibodies.

The analysis membrane 3, furthermore, comprises a chromatographic control area, consisting of the line indicated in figure 2 with the reference 5, comprising M1 aflatoxin secondary antibodies for anti primary antibodies.

The chromatographic control area 5 is suitable for confirming the correctness of the test performed using the chromatographic device 1.

Preferably, the analysis membrane 3 is made of nitrocellulose or the like.

The chromatographic device 1 also comprises a first absorption membrane 6 having a portion suitable for being immersed in the mix obtained from the competition phase D.

In particular, the first absorption membrane 6 is composed of a strip with substantially elongated shape and is fastened to the support element 1 by gluing or mechanical fastening with one of the extremities arranged in the proximity of one of the extremities of the analysis membrane 3.

The first absorption membrane 6 is preferably made of glass wool if necessary treated with adsorbing agents.

Usefully, the chromatographic device 1 can be provided with an additional absorption membrane 7 arranged between the first absorption membrane 6 and the analysis membrane 3 and suitable for increasing the absorption capacity of the mix.

The use of a chromatographic device 1 without the additional absorption membrane 7 cannot however be ruled out.

The additional absorption membrane 7 is preferably made of glass wool if necessary treated with adsorbing agents.

The chromatographic device 1, furthermore, comprises a second absorption membrane 8 suitable for absorbing the excess mix.

In particular, the second absorption membrane 8 is composed of a strip with substantially elongated shape, fastened to the support element 1 by gluing or mechanical fastening.

The second absorption membrane 8 has one of its extremities arranged in the proximity of the extremity of the analysis membrane 3 opposite the first absorption membrane 6.

The second absorption membrane 8 is preferably made of cellulose if necessary treated with adsorbing agents.

Usefully, the first absorption membrane 6, the additional absorption membrane 7, the analysis membrane 3 and the second absorption membrane 8 are arranged along the support element 2 in sequence the one to the other and with their longitudinal axes aligned the one with the other.

The mix obtained from the competition phase D flows from the first absorption membrane 6 towards the second absorption membrane 8, in the direction indicated in figure 2 by the arrow 9, crossing the chromatographic test area 4 and the chromatographic control area 5.

Advantageously, the chromatographic device 1 according to the invention can be fitted in a specific plastic container 10 suitable for protecting the device itself from knocks and for making it easier to use.

A particular but not sole embodiment of such container is shown in the figures 3 and 4.

It has in fact been ascertained how the described invention achieves the proposed objects.

In particular, the fact is underlined that the method according to the invention can be used for qualitative analyses, i.e., aimed at detecting the presence or not of even small quantities of M1 aflatoxin in the milk or in the milk by-products, above or below one or more predetermined thresholds.

In particular, such thresholds can be modulated during the production stage or directly by the user by regulating the quantity of reactants used.

Furthermore, the method according to the invention can be used for accurate quantitative analyses, i.e., aimed at precisely measuring the quantity of M1 aflatoxin in the milk or in the milk by-products.

Advantageously, with the method according to the invention, it is not necessary to operate at controlled temperature, but it is possible to work at ambient temperature.

Consequently, no additional devices have to be used such as incubators or the like which complicate the analysis operations and increase the total costs.

The method according to the invention allows quickly performing the analyses in less than 30 minutes.

A further advantage is the fact that the method according to the invention does not require the use of toxic or in any way harmful reactants.

Furthermore, the method according to the invention can be implemented at a low cost, inasmuch as it does not require complex instruments, such as e.g. high-pressure instruments or the sophisticated fluorescence detectors used in an HPLC system.

A further advantage of the method according to the invention is being able to operate on a broad range of dairy product matrices.

The systems of known type, furthermore, commonly have three reaction points, T1, T2 and C, i.e., test 1, test 2 and Control.

The method according to the invention is technically simpler inasmuch as the lateral-flow device used comprises just two reaction points on the analysis membrane of the M1 aflatoxin, i.e., a chromatographic test area and a chromatographic control area.

This innovative two-line system makes the assay simpler and therefore more easily reproducible and available, depending advantageously on one less variable (the third line in systems of known type).

This greater simplicity also translates into lower production costs and more immediate reading on the part of the operator, using both electronic readers and performing visual readings without the aid of external readers, thus also allowing the visual reading (without reader) of a datum of qualitative type or an estimate of a quantitative datum.

Finally, the method according to the invention comprises easy-to-implement phases.

## Claims

1. Method (M) for analysing M1 aflatoxins in milk and milk by-products, **characterised by** the fact that it comprises:
- at least a homogenization phase (B) of a milk or milk by-product sample to be analysed;
- at least an extraction phase (C) from said homogenized sample of an extract containing M1 aflatoxin;
- at least a competition phase (D) between said extract and an antibody-dye conjugate for obtaining a mix to be analysed wherein the reaction between said M1 aflatoxin and said antibody-dye conjugate is at least partially completed;
- at least a chromatography phase (E) of said mix by means of at least a chromatographic device (1) of the type of a lateral-flow device;
- at least a qualitative and/or quantitative reading phase (F) of the results found by said chromatographic device (1), wherein said qualitative reading comprises the detection of the presence or not in said sample of a quantity of M1 aflatoxin higher than at least a pre-determined minimum threshold level, and wherein said quantitative reading comprises the measurement of the quantity of M1 aflatoxin present in said sample.

2. Method (M) according to the claim 1, **characterised by** the fact that said homogenization phase (B) comprises at least a grinding phase (B1) for a pre-determined time of said sample, if said sample is a solid sample.

3. Method (M) according to the claim 2, **characterised by** the fact that said pre-determined grinding time is equal to 30 seconds.

4. Method (M) according to one or more of the preceding claims, **characterised by** the fact that said homogenization phase (B) comprises at least a mixing phase (B2) for a pre-determined time of said sample, if said sample is a liquid sample.

5. Method (M) according to one or more of the claims 2 or 3, **characterised by** the fact that said extraction phase (C) comprises at least a dilution phase (C1) of said homogenized sample by means of a pre-defined quantity of water or water solution.

6. Method (M) according to one or more of the claims from 2 to 5, **characterised by** the fact that said extraction phase (C) comprises at least a centrifugation phase (C2) of said homogenized sample.

7. Method (M) according to one or more of the preceding claims, **characterised by** the fact that said competition phase (D) comprises placing a pre-defined quantity of the extract inside at least one container containing said antibody-dye conjugate and waiting for a pre-determined waiting time at ambient temperature.

8. Method (M) according to one or more of the preceding claims, **characterised by** the fact that said competition phase (D) comprises placing a pre-defined quantity of the extract inside a container containing said antibody-dye conjugate and an organic or inorganic saline solution and waiting for a pre-determined waiting time at ambient temperature.

9. Method according to one or more of the preceding claims, **characterised by** the fact that said dye is selected from the group comprising colloidal gold, fluorescent dyes, Latex or the like.

10. Method according to one or more of the preceding claims, **characterised by** the fact that said antibody-dye conjugate is in the liquid form or in the freeze-dried form.

11. Method (M) according to one or more of the claims from 7 to 10, **characterised by** the fact that said chromatography phase (E) comprises introducing said chromatographic device (1) inside said container and waiting for a pre-determined time.

12. Method (M) according to one or more of the preceding claims, **characterised by** the fact that said reading phase (F) comprises reading the presence and/or the quantity of M1 aflatoxin found by said chromatographic device (1) by means of a flat scanner.

13. Method (M) according to one or more of the preceding claims, **characterised by** the fact that said chromatographic device (1) comprises at least an analysis membrane (3) of said mix obtained from the competition phase, wherein said analysis membrane (3) comprises at least a chromatographic test area (4) having reactants able, during their passage, to fix the antibody molecules which have not reacted with said extract or which have reacted partially with said extract in said competition phase (D).

14. Method (M) according to the claim 13, **characterised by** the fact that said reactants comprise a protein toxin conjugate able to fix primary antibodies.

15. Method (M) according to the claim 13 or 14, **characterised by** the fact that said analysis membrane (3) comprises at least a chromatographic control area (5) comprising M1 aflatoxin secondary antibodies for anti primary antibodies.

## Patentansprüche

1. Verfahren (M) zur Analyse von M1-Aflatoxinen in Milch und Milch-Nebenprodukten, **dadurch gekennzeichnet, dass** es umfasst:
- zumindest eine Homogenisiertingsphase (B) einer zu analysierenden Probe von Milch oder eines Milch-Nebenprodukts;
- zumindest eine Extraktionsphase (C) dieser homogenisierten Probe eines Extraktes, der M1-Aflatoxin enthält;
- zumindest eine Konkurrenzphase (D) zwischen dem Extrakt und einem Antikörper-Farbstoff-Konjugat, um eine zu analysierende Mischung zu erhalten, wobei die Reaktion zwischen dem M1-Aflatoxin und dem Antikörper-Farbstoff-Konjugat zumindest teilweise abgeschlossen ist;
- zumindest eine Chromatographie-Phase (E) dieser Mischung mittels zumindest eines Chromatographiegeräts (1) vom Typ eines Seitenfluss-Geräts;
- zumindest eine qualitative und/oder quantitative Auslesephase (F) der Resultate, die von dem Chromatographie-Gerät (1) ermittelt wurden, wobei das qualitative Auslesen die Detektion der Anwesenheit oder Nicht-Anwesenheit eine Menge von M1-Aflatoxin umfasst, die höher als zumindest ein vorbestimmtes Minimal-Schwellenniveau ist, und wobei das quantitative Auslesen die Messung der in der Probe vorhandenen Menge von M1-Aflatoxin umfasst.

2. Verfahren (M) nach Anspruch 1, **dadurch gekennzeichnet, dass** die Homogenisierungsphase (B) zumindest eine Mahlphase (B1) für eine vorbestimmte Zeit der Probe umfasst, falls die Probe eine feste Probe ist.

3. Verfahren (M) nach Anspruch 2, **dadurch gekennzeichnet, dass** das die vorbestimmte Mahlzeit gleich 30 Sekunden ist.

4. Verfahren (M) nach einem oder mehreren der vorgenannten Ansprüche, **dadurch gekennzeichnet, dass** die Homogenisieningsphase (B) zumindest eine Mischphase (B2) für eine vorbestimmte Zeit der Probe umfasst, wenn die Probe eine flüssige Probe ist.

5. Verfahren (M) nach Anspruch 2 oder 3, **dadurch gekennzeichnet, dass** die Extraktionsphase (C) zumindest eine Verdünnungsphase (C1) der homogenisierten Probe mittels einer vorbestimmten Menge von Wasser oder wässrigen Lösung umfasst.

6. Verfahren (M) nach einem oder mehreren der Ansprüche 2 bis 5, **dadurch gekennzeichnet, dass** die Extraktionsphase (C) zumindest eine Zentrifugierphase (C2) der homogenisierten Probe umfasst.

7. Verfahren (M) nach einem oder mehreren der vorgenannten Ansprüche, **dadurch gekennzeichnet, dass** die Konkurrenzphase (D) es umfasst, eine vorbestimmte Menge von Extrakt in zumindest einen Behälter zu platzieren, der das Antikörper-Farbstoff-Konjugat enthält, und für eine vorbestimmte Wartezeit bei Umgebungstemperatur zu warten.

8. Verfahren (M) nach einem oder mehreren der vorgenannten Ansprüche, **dadurch gekennzeichnet, dass** die Konkurrenzphase (D) es umfasst, eine vorbestimmte Menge des Extrakts in einen Behälter zu platzieren, der das Antikörper-Farbstoff-Konjugat und eine organische oder anorganische Salzlösung enthält, und für eine vorbestimmte Wartezeit bei Umgebungstemperatur zu warten.

9. Verfahren (M) nach einem oder mehreren der vorgenannten Ansprüche, **dadurch gekennzeichnet, dass** der Farbstoff ausgewählt ist aus der Gruppe umfassend koloidales Gold, fluoreszierende Farbstoffe, Latex oder dergleichen.

10. Verfahren (M) nach einem oder mehreren der vorgenannten Ansprüche, **dadurch gekennzeichnet, dass** das Antikörper-Farbstoff-Konjugat in flüssiger Form oder in gefriergetrockneter Form vorliegt.

11. Verfahren (M) nach einem oder mehreren der Ansprüche 7 bis 10, **dadurch gekennzeichnet, dass** die Chromatographiephase (E) es umfasst, das Chromatographiegerät (1) in den Behälter einzuführen und für eine vorbestimmte Zeit zu warten.

12. Verfahren (M) nach einem oder mehreren der vorgenannten Ansprüche, **dadurch gekennzeichnet, dass** die Auslesephase (F) es umfasst, die Anwesenheit und/oder die Menge von M1-Aflatoxin, die von dem Chromatographiegerät (1) gefunden wurde, mittels eines Flachscanners auszulesen.

13. Verfahren (M) nach einem oder mehreren der vorgenannten Ansprüche, **dadurch gekennzeichnet, dass** das Chromatographiegerät (1) zumindest eine Analysemembran (3) der Mischung, die aus der Konkurrenzphase erhalten wurde, umfasst, wobei die Analysemembran (3) zumindest einen Chromatographie-Testbereich (4) umfasst, der Reaktionspartner aufweist, die in der Lage sind, während ihres Durchgangs die Antikörper-Moleküle zu fixieren, die mit dem Extrakt nicht reagiert haben oder die teilweise mit dem Extrakt in der Konkurrenzphase (D) reagiert haben.

14. Verfahren (M) nach Anspruch 13, **dadurch gekennzeichnet, dass** die Reaktionspartner ein Protein-Toxin-Konjugat aufweisen, die in der Lage sind, primäre Antikörper zu fixieren.

15. Verfahren (M) nach Anspruch 13 oder 14, **dadurch gekennzeichnet, dass** die Analysemembran (3) zumindest einen chromatographischen Kontrollbereich (5) umfasst, der M1-Aflatoxin-Sekundär-Antikörper für Anti-Primär-Antikörper aufweist.

## Revendications

1. Procédé (M) d'analyse d'aflatoxines M1 dans le lait et dans des produits dérivés du lait, **caractérisé par le fait qu'**il comprend :
- au moins une phase d'homogénéisation (B) d'un échantillon de lait ou de produit dérivé du lait devant être analysé ;
- au moins une phase d'extraction (C), à partir dudit échantillon homogénéisé, d'un extrait contenant de l'aflatoxine M1;
- au moins une phase de concurrence (D) entre ledit extrait et un conjugué anticorps-colorant en vue de l'obtention d'un mélange devant être analysé, la réaction entre ladite aflatoxine M1 et ledit conjugué anticorps-colorant étant au moins partiellement effectuée ;
- au moins une phase de chromatographie (E) dudit mélange au moyen d'au moins un dispositif de chromatographie (1) du type dispositif à flux latéral ;
- au moins une phase de lecture qualitative et/ou quantitative (F) des résultats trouvés par ledit dispositif de chromatographie (1), ladite lecture qualitative comprenant la détection de la présence ou non, dans ledit échantillon, d'une quantité d'aflatoxine M1 supérieure à au moins un niveau de seuil minimal prédéterminé, et ladite lecture quantitative comprenant la mesure de la quantité d'aflatoxine M1 présente dans ledit échantillon.

2. Procédé (M) selon la revendication 1, **caractérisé par le fait que** ladite phase d'homogénéisation (B) comprend au moins une phase de broyage (B1) dudit échantillon pendant une durée prédéterminée, lorsque ledit échantillon est un échantillon solide.

3. Procédé (M) selon la revendication 2, **caractérisé par le fait que** ladite durée de broyage prédéterminée est égale à 30 secondes.

4. Procédé (M) selon une ou plusieurs des revendications précédentes, **caractérisé par le fait que** ladite phase d'homogénéisation (B) comprend au moins une phase de mélange (B2) dudit échantillon pendant une durée prédéterminée, lorsque ledit échantillon est un échantillon liquide.

5. Procédé (M) selon une ou plusieurs des revendications 2 ou 3, **caractérisé par le fait que** ladite phase d'extraction (C) comprend au moins une phase de dilution (C1) dudit échantillon homogénéisé au moyen d'une quantité prédéfinie d'eau ou d'une solution aqueuse.

6. Procédé (M) selon une ou plusieurs des revendications 2 à 5, **caractérisé par le fait que** ladite phase d'extraction (C) comprend au moins une phase de centrifugation (C2) dudit échantillon homogénéisé.

7. Procédé (M) selon une ou plusieurs des revendications précédentes, **caractérisé par le fait que** ladite phase de concurrence (D) consiste à placer une quantité prédéfinie de l'extrait à l'intérieur d'au moins un récipient contenant ledit conjugué anticorps-colorant, et à attendre à la température ambiante pendant une durée d'attente prédéterminée.

8. Procédé (M) selon une ou plusieurs des revendications précédentes, **caractérisé par le fait que** ladite phase de concurrence (D) consiste à placer une quantité prédéfinie de l'extrait à l'intérieur d'au moins un récipient contenant ledit conjugué anticorps-colorant et une solution saline organique ou inorganique, et à attendre à la température ambiante pendant une durée d'attente prédéterminée.

9. Procédé selon une ou plusieurs des revendications précédentes, **caractérisé par le fait que** ledit colorant est choisi dans le groupe comprenant de l'or colloïdal, des colorants fluorescents, du latex, ou similaires.

10. Procédé selon une ou plusieurs des revendications précédentes, **caractérisé par le fait que** ledit conjugué anticorps-colorant est sous la forme liquide ou sous la forme lyophilisée.

11. Procédé (M) selon une ou plusieurs des revendications 7 à 10, **caractérisé par le fait que** ladite phase de chromatographie (E) consiste à introduire ledit dispositif de chromatographie (1) à l'intérieur dudit récipient et à attendre pendant une durée prédéterminée.

12. Procédé (M) selon une ou plusieurs des revendications précédentes, **caractérisé par le fait que** ladite phase de lecture (F) consiste à lire au moyen d'un scanner à plat la présence et/ou la quantité d'aflatoxine M1 trouvé(e)s par ledit dispositif de chromatographie (1).

13. Procédé (M) selon une ou plusieurs des revendications précédentes, **caractérisé par le fait que** ledit dispositif de chromatographie (1) comprend au moins une membrane (3) d'analyse dudit mélange obtenu à la suite de la phase de concurrence, ladite membrane d'analyse (3) comprenant au moins une zone d'essai chromatographique (4) comportant des réactifs capables, pendant leur passage, de fixer les molécules d'anticorps qui, dans ladite phase de concurrence (D), n'ont pas réagi avec ledit extrait, ou qui ont réagi partiellement avec ledit extrait.

14. Procédé (M) selon la revendication 13, **caractérisé par le fait que** lesdits réactifs comprennent un conjugué de toxine protéine capable de fixer des anticorps primaires.

15. Procédé (M) selon la revendication 13 ou 14, **caractérisé par le fait que** ladite membrane d'analyse (3) comprend au moins une zone de contrôle chromatographique (5) comportant des anticorps secondaires d'aflatoxine M1 pour des anti- anticorps primaires.
